# EUROPEAN PATENT APPLICATION

(11) **EP 3 604 282 A1**
(43) Date of publication of application: **05.02.2020**
(21) Application number: 18775452.8
(22) Date of filing: 23.03.2018
(51) Int. Cl.: C07D 231/14, A61K 31/415, A61P 31/04

(54) **METHOD FOR PRODUCING HALOGEN-CONTAINING PYRAZOL CARBOXYLIC ACID AND INTERMEDIATE THEREOF**

(30) Priority: 27.03.2017 JP 2017061688
(71) Applicant: AGC Inc., Chiyoda-ku Tokyo 100-8405 (JP)
(72) Inventor: ISHIBASHI, Yuichiro, Tokyo 100-8405 (JP)
(74) Representative: dompatent von Kreisler Selting Werner - Partnerschaft von Patent- und Rechtsanwälten mbB
(86) International application number: PCT/JP2018/011582
(87) International publication number: WO 2018/180943

(57) **Abstract**

The present invention provides a method capable of more simply and efficiently producing halogen-containing pyrazolecarboxylic acids and intermediates thereof, which are useful as pharmaceutical or agrochemical intermediates, in a manner suitable for industrial production.

The present invention relates to a method of producing a compound represented by the formula (b), which comprises reacting a compound represented by the formula (a) with a halogenating agent selected from the group consisting of a compound represented by X₂ (formula (1)), a compound represented by SOX₂ (formula (2)), and a compound represented by SO₂X₂ (formula (3)) to obtain the compound represented by the formula (b):

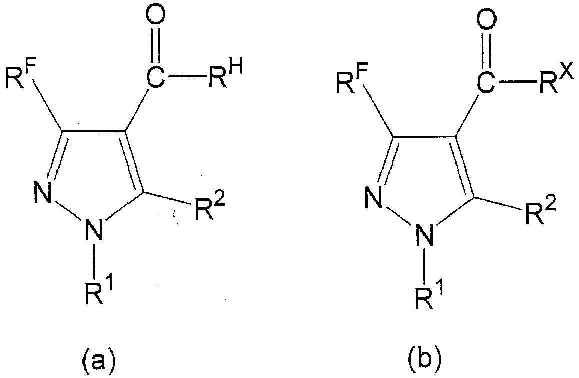

formula (1) X₂

formula (2) SOX₂

formula (3) SO₂X₂

wherein each symbol is as described in the description.

## Description

### Technical Field

The present invention relates to a method of producing halogen-containing pyrazolecarboxylic acids (preferably fluorine-containing pyrazolecarboxylic acids) and an intermediate thereof, which are useful as pharmaceutical or agrochemical intermediates.

### Background Art

Halogen-containing pyrazolecarboxylic acids such as 3-difluoromethyl-1-methyl-1H-pyrazole-4-carboxylic acid and the like are intermediates useful for pyrazolylcarboxanilide fungicides.

Patent Document 1 discloses a method of reacting a compound represented by the following formula (a¹) with sodium hypochlorite in the presence of water to obtain a compound represented by the formula (c¹).

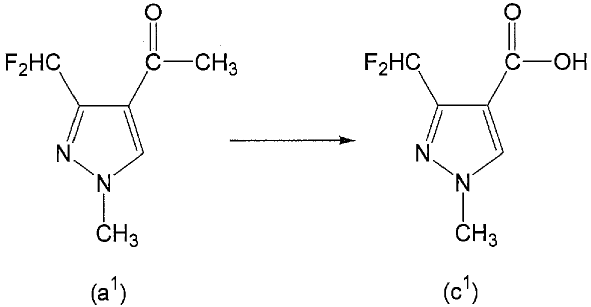

### Document List

### Patent Document

Patent Document 1: WO 2016/152886

### Summary of the Invention

### Problems to be Solved by the Invention

In the method using sodium hypochlorite, which is disclosed in Patent Document 1, an aqueous solution containing sodium hypochlorite is generally used.

In an aqueous solution mentioned above, the concentration of sodium hypochlorite cannot be increased, and therefore, a large amount of aqueous solution is required, which leads to additional work for wastewater treatment after reaction. Hence, use of an aqueous solution is not desirable from industrial aspect.

The aim of the present invention is to provide a method capable of more simply and efficiently producing halogen-containing pyrazolecarboxylic acids and intermediates thereof, which are useful as pharmaceutical or agrochemical intermediates, in a manner suitable for industrial production.

### Means of Solving the Problems

The present inventors have conducted intensive studies, and found that the above-mentioned problems can be solved by use of a specific halogenating agent.

Accordingly, the present invention encompasses the following inventions.
(1) a method of producing the below-mentioned compound represented by the formula (b), which comprises reacting the below-mentioned compound represented by the formula (a) with a halogenating agent selected from the group consisting of the below-mentioned compound represented by the formula (1), the below-mentioned compound represented by the formula (2), and the below-mentioned compound represented by the formula (3) to obtain the compound represented by the formula (b).
(2) The method of (1), wherein the reaction of the compound represented by the formula (a) with the halogenating agent is carried out under an acidic or neutral condition.
(3) The method of (1) or (2), wherein X is a chlorine atom.
(4) The method of any of (1) to (3), wherein the halogenating agent is chlorine.
(5) The method of any of (1) to (4), wherein the reaction of the compound represented by the formula (a) with the halogenating agent is carried out in the presence of an organic solvent.
(6) The method of (5), wherein the organic solvent is a haloalkane.
(7) The method of any of (1) to (6), wherein R^{X} is CH₂X or CHX₂.
(8) A method of producing the below-mentioned compound represented by the formula (c), which comprises subjecting the compound represented by the formula (b) obtained by the method of any of (1) to (7) to hydrolysis to obtain the compound represented by the formula (c).
(9) The method of (8), wherein the hydrolysis of the compound represented by the formula (b) is carried out in the presence of a salt of halogen oxoacid, and then the resulting compound is reacted with an acid to obtain the compound represented by the formula (c).
(10) The method of (8), wherein the hydrolysis of the compound represented by the formula (b) is carried out in the presence of an alkali, and then the resulting compound is reacted with an acid to obtain the compound represented by the formula (c).
(11) The below-mentioned compound represented by the formula (b) .
(12) The compound of (11), wherein R^{X} is CH₂X or CHX₂.

### Effect of the Invention

The present invention can provide a method capable of more simply and efficiently producing halogen-containing pyrazolecarboxylic acids and intermediates thereof, which are useful as pharmaceutical or agrochemical intermediates, in a manner suitable for industrial production.

### Description of Embodiments

The present invention is explained below in detail. As used herein, numerical range shown by using "to" means the range including the numerical values described before and after "to" as minimum and maximum values.

The present invention a method of producing a compound represented by the formula (b) (hereinafter, referred to as "compound (b)"), which comprises reacting a compound represented by the formula (a) (hereinafter, referred to as "compound (a)") with a halogenating agent selected from the group consisting of a compound represented by the formula (1) (hereinafter, referred to as "compound (1)"), a compound represented by the formula (2) (hereinafter, referred to as "compound (2)"), and a compound represented by the formula (3) (hereinafter, referred to as "compound (3)") to obtain compound (b) .

As mentioned below, compound (b) is useful as an intermediate for the below-mentioned compound represented by the formula (c) (hereinafter, referred to as "compound (c)"). When compound (c) is produced via an intermediate, isolation and purification of the intermediate enables the production of compound (c) with higher purity.

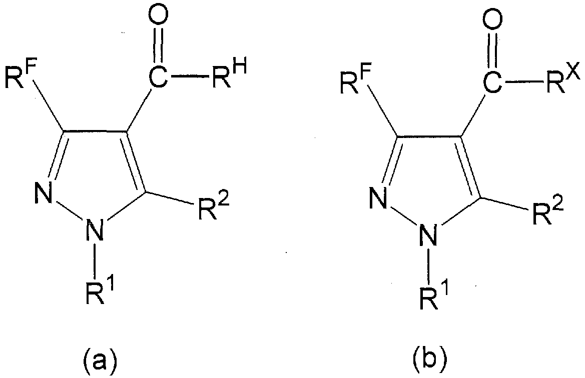

formula (1) X₂

formula (2) SOX₂

formula (3) SO₂X₂.

R¹ is an alkyl group having1 to 3 carbon atoms, preferably a methyl group.

R² is a hydrogen atom or a halogen atom. Specific examples of the halogen atom include a fluorine atom, a chlorine atom, a bromine atom and an iodine atom. R² is preferably a hydrogen atom.

R^{F} is a haloalkyl group having 1 to 3 carbon atoms. The haloalkyl group means a group wherein one or more hydrogen atoms of the alkyl group are replaced with halogen atoms. Specific examples of the halogen atom include a fluorine atom, a chlorine atom, a bromine atom and an iodine atom.

Specific examples of R^{F} include monohalomethyl groups (e.g., a fluoromethyl group, a chloromethyl group), dihalomethyl groups (e.g., a difluoromethyl group, a dichloromethyl group), trihalomethyl groups (e.g., a trifluoromethyl group, a dichlorofluoromethyl group, a chlorodifluoromethyl group), monohaloethyl groups (e.g., a 2-fluoroethyl group, a 2-chloroethyl group) and dihaloethyl groups (e.g., a 2,2-difluoroethyl group, a 2,2-dichloroethyl group).

R^{F} is preferably a monohalomethyl group, a dihalomethyl group or a trihalomethyl group, more preferably a dihalomethyl group or a trihalomethyl group, still more preferably a difluoromethyl group, a dichlorofluoromethyl group or a chlorodifluoromethyl group, particularly preferably a difluoromethyl group.

R^{H} is CₘH₂ₘ₊₁, R^{X} is CₘH₂ₘ₊₁₋ₙXₙ, m is an integer of 1 to 3, and n is an integer of 1 to 2m+1.

m is preferably 1 or 2, more preferably 1. That is to say, R^{H} is preferably CH₃ or C₂H₅, more preferably CH₃.

When m is 1, then n is 1 to 3, preferably 1 or 2, more preferably 2, from the aspect of reduction in waste amount and cost saving, due to a small amount of the halogenating agent to be used. That is to say, when m is 1, R^{X} is CH₂X, CHX₂ or CX₃, preferably CH₂X or CHX₂, more preferably CHX₂.

When m is 2, then n is an integer of 1 to 5, and when m is 3, then n is an integer of 1 to 7.

X is a chlorine atom, a bromine atom or an iodine atom, preferably a chlorine atom.

Compound (1) is preferably Cl₂ or Br₂, more preferably Cl₂.

Compound (2) is preferably SOCl₂ or SOBr₂, more preferably SOCl₂.

Compound (3) is preferably SO₂Cl₂ or SO₂Br₂, more preferably SO₂Cl₂.

The halogenating agent is preferably compound (1), more preferably Cl₂.

The reaction of compound (a) with a halogenating agent can be carried out in the presence or absence of an organic solvent. When the halogenating agent is compound (1), since the contact efficiency to compound (a) is higher, the reaction is preferably carried out in the presence of an organic solvent. The organic solvent is preferably a solvent inert to the halogenating agent, and examples thereof include haloalkanes such as chloroform, methylene chloride, 1,2-dichloroethane and the like; ethers such as diethyl ether, tetrahydrofuran, dioxane, 1,2-dimethoxyethane and the like; aromatic hydrocarbons such as benzene, toluene, xylene, mesitylene and the like; halogenated aromatic hydrocarbons such as monochlorobenzene, dichlorobenzene and the like; and aliphatic hydrocarbons such as hexane, heptane, octane, cyclohexane and the like. Among them, preferred is a haloalkane, and more preferred is chloroform, from the aspect of conversion of the reaction.

As another preferable embodiment of the organic solvent, protic organic solvents can also be used. Specific examples of the protic organic solvent include alkyl carboxylic acids such as acetic acid, propionic acid, butanoic acid, hexanoic acid, heptanoic acid, octanoic acid, pivalic acid, 3-methylbutanoic acid and the like. The amount of the organic solvent to be used is preferably 30-fold mass or less, more preferably 2 to 20-fold mass, still more preferably 2 to 10-fold mass, relative to compound (a), from the aspect of economic efficiency.

The moisture content in the organic solvent is desirably less, from the aspect of easy purification on recovery and recycling of the organic solvent, and reduction in wastewater amount. Specifically, it is preferably 10 mass% or less, more 5 mass% or less, relative to the organic solvent.

The moisture content in the system of the reaction of compound (a) with a halogenating agent is desirably less, preferably 10 mass% or less, more preferably 5 mass% or less, still more preferably 1 mass% or less, from the aspect of reduction in wastewater amount.

The amount of the halogenating agent to be used is preferably 1 mol equivalent or more relative to compound (a) from the aspect of conversion of the reaction, and preferably 12 mol equivalent or less relative to compound (a) from the aspect of inhibition of side reaction. The number of X (i.e., n) in R^{X}(CₘH₂ₘ₊₁₋ₙXₙ) depends on the amount of the halogenating agent to be used. For example, when the halogenating agent is Cl₂, and R^{H} is a methyl group, then the amount of the halogenating agent to be used is preferably 1 to 2 mol equivalent for conversion to CH₂Cl, 2 to 4 mol equivalent for conversion to CHCl₂, and 3 to 9 mol equivalent for conversion to CCl₃.

The halogenating agent may be used alone or in combination of two or more kinds thereof.

The reaction of compound (a) with a halogenating agent is preferably carried out by mixing them. As a method of mixing compound (a) and a halogenating agent, for example, a method of introducing a halogenating agent into a solution containing compound (a) and an organic solvent can be employed.

In mixing compound (a) and a halogenating agent, both may be mixed in a batch, or one of compound (a) and a halogenating agent may be mixed to the other in divided portions.

The halogenating agent may be used directly or in the form of a solution in a solvent (for example, chloroform).

The reaction temperature in the reaction of compound (a) with the above-mentioned halogenating agent is preferably 0 to 100°C, more preferably 5 to 50°C, particularly preferably 10 to 30°C, from the aspect of efficient reaction progress by inhibition of side reaction.

As a method of isolating compound (b) from the system after the completion of the reaction, for example, evaporation under reduced pressure, solvent extraction and crystallization can be employed. Compound (b) may be purified by column chromatography and the like, if necessary.

The reaction of compound (a) with the above-mentioned halogenating agent is preferably carried out under an alkali-free condition. In other words, the reaction of compound (a) with the above-mentioned halogenating agent is preferably carried out under an acidic or neutral condition.

Specific examples of the alkali include sodium hydroxide and potassium hydroxide.

The acidic or neutral condition means that the pH of the solution, which is prepared by adding a part (1 ml) taken out of the reaction system to water (10 ml), is 7 or below.

The present invention also provides a method of producing compound (c), which comprises subjecting compound (b) obtained by the above-mentioned method to hydrolysis to obtain compound (c) .

Compound (b) obtained by the above-mentioned method may be subjected to the production method of compound (c), after isolation or sequentially without isolation. When compound (b) is not isolated, the compound may be sequentially subjected to the production method of compound (c) without removal of the halogenating agent remaining in the reaction system. The "without removal of the halogenating agent" means, for example, that, when chlorine gas is used as a halogenating agent, the chlorine gas is not purged.

The production method of compound (c) may be sequentially started before the completion of the production method of compound (b). For example, when the conversion of compound (a) to compound (b) proceeds to about 80% or more in the production method of compound (b), the hydrolysis of compound (b) may be started by adding a salt of halogen oxoacid or an alkali to the reaction system as mentioned below, without isolation of compound (b). 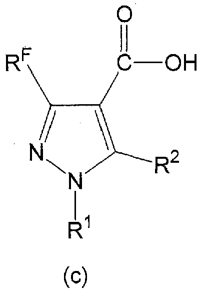 wherein R¹, R² and R^{F} are as defined above.

The first preferable embodiment of the production method of compound (c) is an embodiment of subjecting compound (b) to hydrolysis in the presence of a salt of halogen oxoacid, and then reacting the resulting compound with an acid to obtain compound (c).

Specific examples of the salt of halogen oxoacid include alkali metal salts of halogen oxoacid, and alkaline-earth metal salts of halogen oxoacid, and more specific examples thereof include potassium salts of halogen oxoacid, halogen oxoacids of sodium salt, and calcium salts of halogen oxoacid. The salt of halogen oxoacid is preferably a potassium salt of halogen oxoacid, or a sodium salt of halogen oxoacid, more preferably a sodium salt of halogen oxoacid.

Specific examples of the halogen oxoacid include halous acid, hypohalous acid, halogen acid and perhalogen acid, and more specific examples thereof include chlorous acid, hypochlorous acid, chloric acid, perchloric acid, bromous acid, hypobromous acid, bromic acid and perbromic acid. The halogen oxoacid is preferably hypohalous acid, more preferably hypochlorous acid.

The salt of halogen oxoacid is preferably sodium hypochlorite.

The amount of the salt of halogen oxoacid to be used is preferably 0.8 mol equivalent or more relative to compound (b) from the aspect of inhibition of side reaction, and preferably 2.0 mol equivalent or less, more preferably 0.8 to 2.0 mol equivalent, relative to compound (b), from the aspect of conversion of the reaction.

The reaction of compound (b) with a salt of halogen oxoacid is preferably carried out by mixing them. As a method of mixing compound (b) and a salt of halogen oxoacid, for example, a method of adding dropwise a solution containing compound (b) to an aqueous solution containing a salt of halogen oxoacid can be employed.

The reaction temperature in the hydrolysis of compound (b) is preferably 0 to 100°C, more preferably 0 to 30°C, from the aspect of efficient reaction progress by inhibition of side reaction.

The hydrolysis of compound (b) may be carried out in the presence of a solvent (for example, water, an organic solvent).

Next, the hydrolysate of compound (b) is reacted with an acid to obtain compound (c). For example, when an aqueous sodium hypochlorite solution is used as a salt of halogen oxoacid, a compound represented by the following formula is obtained as the hydrolysate of compound (b).

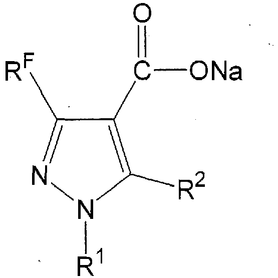

The hydrolysate of compound (b) may be reacted with an acid after isolation or sequentially without isolation. As an isolation method, a separation method can be employed.

Examples of the acid include sulfuric acid, hydrogen chloride, hydrochloric acid and nitric acid.

As a method of reacting the hydrolysate of compound (b) with an acid, a method of mixing the hydrolysate of compound (b) and an acid can be employed.

The second preferable embodiment of the production method of compound (c) is an embodiment of subjecting compound (b) to hydrolysis in the presence of an alkali, and then reacting the resulting compound with an acid to obtain compound (c).

Specific examples of the alkali include lithium hydroxide, sodium hydroxide, potassium hydroxide, sodium methoxide, sodium ethoxide, sodium-tert-butoxide, potassium methoxide, potassium ethoxide and potassium-tert-butoxide, and preferred are sodium hydroxide and potassium hydroxide.

The amount of the alkali to be used is preferably 1 mol equivalent or more relative to compound (b) from the aspect of conversion of the reaction, and preferably 2 mol equivalent or less, more preferably 1.0 to 2.0 mol equivalent, relative to compound (b), from the aspect of inhibition of side reaction.

As a method of mixing compound (b) and an alkali, for example, a method of adding dropwise an aqueous solution containing an alkali to a solution containing compound (b) can be employed.

The reaction temperature in the hydrolysis of compound (b) is preferably -10 to 100°C, more preferably -5 to 50°C, particularly preferably 0 to 30°C, from the aspect of efficient reaction progress by inhibition of side reaction.

The hydrolysis of compound (b) may be carried out in the presence of a solvent (for example, water, an organic solvent).

Next, the hydrolysate of compound (b) is reacted with an acid to obtain compound (c). The procedure is the same as explained in the first preferable embodiment.

The method of hydrolyzing compound (b) may be a method other than those explained as in the first and second preferable embodiments, for example, a method using an acid can also be employed.

As a method of isolating compound (c) from the system after the completion of the reaction, for example, solvent extraction and crystallization can be employed.

Compound (c) is useful as intermediates for antimicrobials, fungicides, or bulk pharmaceuticals or agrochemicals, for example, useful as intermediates for the following antimicrobials, fungicides, or bulk pharmaceuticals or agrochemicals.

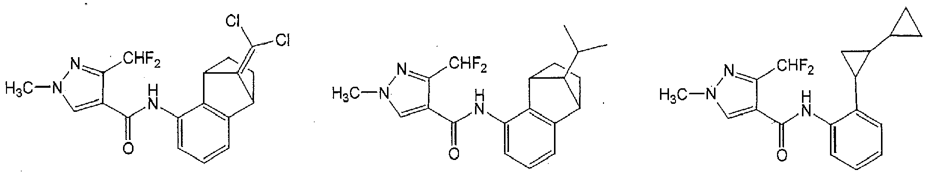

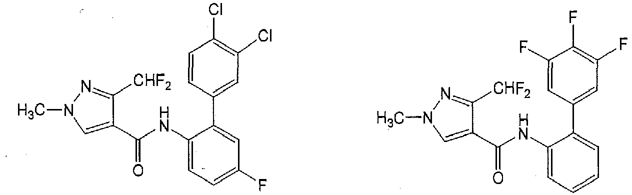

As a method of producing antimicrobials, fungicides, or bulk pharmaceuticals or agrochemicals using compound (c), the method described in Bioorganic & Medicinal Chemistry 24 (2016), p.317-341, or Chem. Rev. 114(2014), p.7079 can be employed. Specifically, for example, a method of reacting compound (c) with thionyl chloride to convert the carboxy group of compound (c) to the corresponding acid chloride group, and then reacting the resulting compound with the following amine compound can be employed.

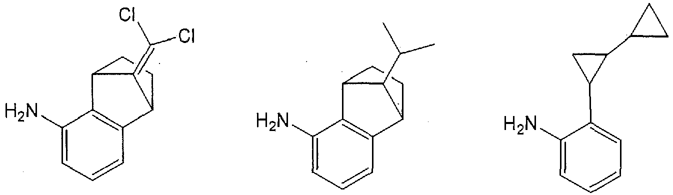

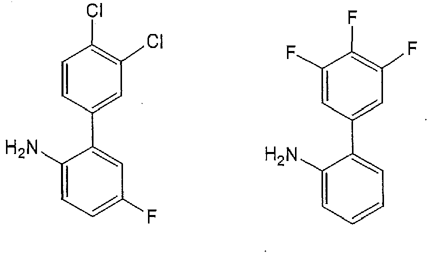

According to the production method of the present invention, the wastewater amount is reduced, and therefore, compound (c) can be more efficiently produced than in conventional art. That is to say, antimicrobials, fungicides, or bulk pharmaceuticals or agrochemicals can be more efficiently produced than in conventional art.

### Examples

The present invention is explained below by referring to Examples, which are not to be construed as limitative.

### [Example 1]

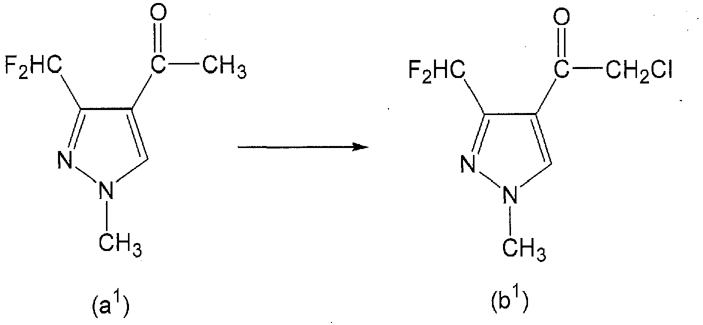

Under nitrogen atmosphere, compound (a¹) (10.0 g) and chloroform (30 ml) were placed in a flask, and chlorine gas (4.07 g) was flowed into the flask at 15°C. The volatile component was evaporated under reduced pressure, and the obtained crude product was purified by column chromatography to give compound (b¹) (yield: 75%).

The NMR data of compound (b¹) was shown below.
¹H-NMR (400MHz,CDCl₃): δ=8.04(s,1H), 7.25-6.89(m,1H), 4.45(s,2H), 4.00(s,3H)
¹⁹F-NMR (400MHz,CDCl₃) δ=-115.63(d,J=54.9Hz,1F)

### [Example 2]

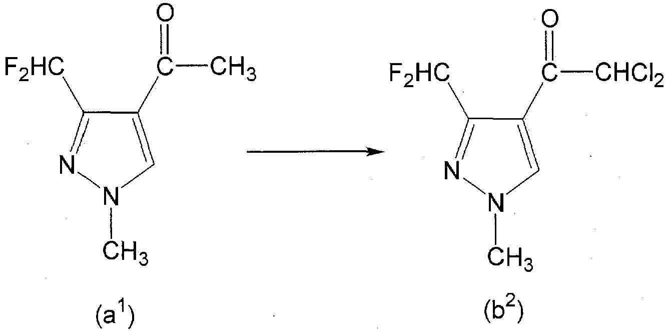

Under nitrogen atmosphere, compound (a¹) (10.0 g) and chloroform (30 ml) were placed in a flask, and chlorine gas (8.14 g) was flowed into the flask at 15°C. The volatile component was evaporated under reduced pressure, and the obtained crude product was purified by column chromatography to give compound (b²) (yield: 80%).

The NMR data of compound (b²) was shown below.
¹H-NMR (400MHz,CDCl₃): δ=8.27(s,1H), 7.10(t,J=53.7Hz,1H), 6.17(s,1H), 4.04(s,3H)
¹⁹F-NMR (400MHz,CDCl₃): δ=-117.33(d,J=53.5Hz,1F)

### [Example 3]

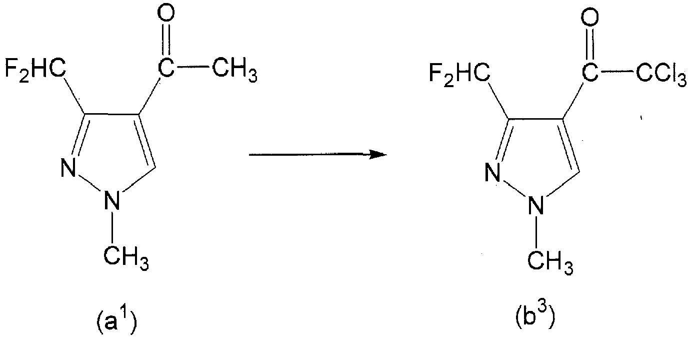

Under nitrogen atmosphere, compound (a¹) (10.0 g) and chloroform (30 ml) were placed in a flask, and chlorine gas (12.21 g) was flowed into the flask at 15°C. The volatile component was evaporated under reduced pressure, and the obtained crude product was purified by column chromatography to give compound (b³) (yield: 80%).

The NMR data of compound (b³) was shown below.
¹H-NMR (400MHz,C₆D₆) δ=8.31(s,1H), 6.95(t,J=53.2Hz,1H), 3.91(s,3H)
¹⁹F-NMR (400MHz, C₆D₆) δ=-116.17(d,J=53.8Hz,1F)

### [Example 4]

Under nitrogen atmosphere, compound (a¹) (1.0 g) and SO₂Cl₂ (2.3 ml) were placed in a flask, and the reaction solution was stirred at 24°C for 10 min. The volatile component was evaporated under reduced pressure, and the obtained crude product was purified by column chromatography to give compound (b²) (yield: 83%).

### [Example 5]

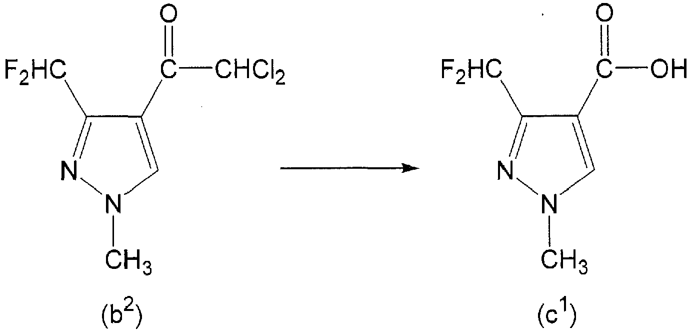

Under nitrogen atmosphere, 11% concentration of aqueous sodium hypochlorite solution (3.1 g) was placed in a flask, and a solution prepared by dissolving compound (b²) (1.0 g) in chloroform (3 ml) was added dropwise to the flask. The reaction solution was stirred at 24°C for 4 hr, 10% concentration of aqueous sodium sulfite solution (5 ml) was added to the flask, and the aqueous phase was recovered by separation operation. The pH of the obtained aqueous phase was adjusted to 3 or below with sulfuric acid, and the precipitated compound (c¹) was collected by filtration to give compound (c¹) (yield: 91%).

### [Example 6]

Under nitrogen atmosphere, a solution prepared by dissolving compound (b²) (1.0 g) in chloroform (3 ml), and water(1 ml) were placed in a flask, and the mixture was cooled in an ice-water bath. While maintaining the flask internal temperature within 0°C to 10°C, 10% concentration of aqueous sodium hydroxide solution (3.2 g) was added dropwise to the flask. At the internal temperature of 0°C or higher and 10°C or lower, the reaction solution was stirred for 2 hr, and the aqueous phase was recovered by separation operation. The pH of the obtained aqueous phase was adjusted to 3 or below with sulfuric acid, and the precipitate was collected by filtration to give compound (c¹) (yield: 43%).

### Industrial Applicability

As is clear from the above results, according to the production method of the present invention, halogen-containing pyrazolecarboxylic acids and intermediates thereof, which are useful as pharmaceutical or agrochemical intermediates, can be produced more simply and efficiently.

This application is based on patent application No. 2017-061688 filed on March 27, 2017 in Japan, the contents of which are encompassed in full herein.

## Claims

1. A method of producing a compound represented by the formula (b), which comprises reacting a compound represented by the formula (a) with a halogenating agent selected from the group consisting of a compound represented by the formula (1), a compound represented by the formula (2) and a compound represented by the formula (3) to obtain the compound represented by the formula (b); 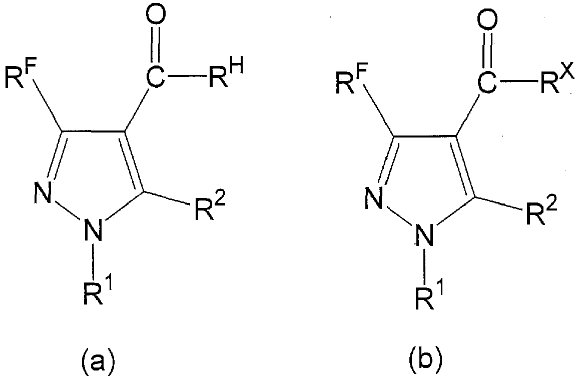
formula (1) X₂
formula (2) SOX₂
formula (3) SO₂X₂
wherein
R¹ is an alkyl group having 1 to 3 carbon atoms,
R² is a hydrogen atom or a halogen atom,
R^{F} is a haloalkyl group having 1 to 3 carbon atoms,
R^{H} is CₘH₂ₘ₊₁,
R^{X} is CₘH₂ₘ₊₁₋ₙXₙ,
X is a chlorine atom, a bromine atom or an iodine atom,
m is an integer of 1 to 3, and
n is an integer of 1 to 2m+1.

2. The method according to claim 1, wherein the reaction of the compound represented by the formula (a) with the halogenating agent is carried out under an acidic or neutral condition.

3. The method according to claim 1 or 2, wherein X is a chlorine atom.

4. The method according to any one of claims 1 to 3, wherein the halogenating agent is chlorine.

5. The method according to any one of claims 1 to 4, wherein the reaction of the compound represented by the formula (a) with the halogenating agent is carried out in the presence of an organic solvent.

6. The method according to claim 5, wherein the organic solvent is a haloalkane.

7. The method according to any one of claims 1 to 6, wherein R^{X} is CH₂X or CHX₂.

8. A method of producing a compound represented by the formula (c), which comprises subjecting the compound represented by the formula (b) obtained by the method according to any one of claims 1 to 7 to hydrolysis to obtain the compound represented by the formula (c); 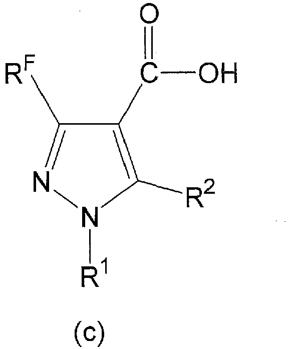 wherein
R¹ is an alkyl group having 1 to 3 carbon atoms,
R² is a hydrogen atom or a halogen atom, and
R^{F} is a haloalkyl group having 1 to 3 carbon atoms.

9. The method according to claim 8, wherein the hydrolysis of the compound represented by the formula (b) is carried out in the presence of a salt of halogen oxoacid, and then the resulting compound is reacted with an acid to obtain the compound represented by the formula (c).

10. The method according to claim 8, wherein the hydrolysis of the compound represented by the formula (b) is carried out in the presence of an alkali, and then the resulting compound is reacted with an acid to obtain the compound represented by the formula (c).

11. A compound represented by the formula (b); 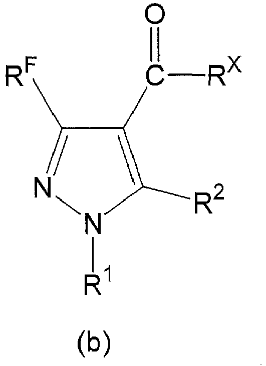 wherein
R¹ is an alkyl group having 1 to 3 carbon atoms,
R² is a hydrogen atom or a halogen atom,
R^{F} is a haloalkyl group having 1 to 3 carbon atoms,
R^{X} is CₘH₂ₘ₊₁₋ₙXₙ,
X is a chlorine atom, a bromine atom or an iodine atom,
m is an integer of 1 to 3, and
n is an integer of 1 to 2m+1.

12. The compound according to claim 11, wherein R^{X} is CH₂X or CHX₂.
